# EUROPEAN PATENT APPLICATION

(11) **EP 4 046 682 A1**
(43) Date of publication of application: **24.08.2022**
(21) Application number: 21158760.5
(22) Date of filing: 23.02.2021
(51) Int. Cl.: A61N 1/375, A61B 17/12

(54) **IMPLANTABLE MEDICAL DEVICE COMPRISING AN ANCHORING DEVICE FOR ANCHORING ON INTRACARDIAC TISSUE**

(71) Applicant: BIOTRONIK SE & Co. KG, 12359 Berlin (DE)
(72) Inventor: HUGHES, Devan, Tualatin, Oregon 97026 (US); LANG, Volker, 12161 Berlin (DE); MUESSIG, Dirk, West Linn, Oregon 97068 (US); KLAUS, Sebastian, 8424 Embrach (CH); HENSCHEL, Martin, 13125 Berlin (DE)
(74) Representative: Biotronik Corporate Services SE

(57) **Abstract**

An implantable medical device (1) comprising a housing (10) and an anchoring device (2) arranged on the housing (10), the anchoring device (2) being configured to anchor the implantable medical device (1) on intra-cardiac tissue. The anchoring device (2) comprises an arrangement of elastic struts (20) for abutment with intra-cardiac tissue, each strut (20) comprising a first end (200) and a second end (201), the struts being connected to the housing (10) at their first ends (200). The anchoring device (2) further comprises a connection structure (21) connecting at least some of the struts (20) of the arrangement of struts (20) at their second ends (201).

## Description

The instant invention generally relates to an implantable medical device and a method for implanting an implantable medical device.

An implantable medical device of this kind may for example be an active implantable medical device (in short AIMD) which comprises electronic circuitry and an energy supply for performing a medical function within a patient's heart, such as a leadless pacemaker for implantation into the heart, for example in the right atrium of the heart.

In recent years, leadless pacemakers have received increasing attention. Leadless pacemakers, in contrast to pacemakers implanted subcutaneously using leads extending transvenously into the heart, avoid leads in that the pacemaker device itself is implanted into the heart, the pacemaker having the shape of a generally cylindrical capsule for implantation into cardiac tissue. Such leadless pacemakers exhibit the inherent advantage of not using leads, which can reduce risks for the patient involved with leads transvenously accessing the heart, such as the risk of pneumothorax, lead dislodgement, cardiac perforation, venous thrombosis and the like.

When placing an implantable medical device such as a leadless pacemaker device in the human heart, one challenge lies in designing an anchoring device allowing a fixation of the implantable medical device on cardiac tissue such that the implantable medical device is securely received and held to the cardiac tissue. For this, the anchoring device on the one hand shall provide for a secure fastening of the implantable medical device on the cardiac tissue, on the other hand however shall avoid a damaging of cardiac tissue and structures within the cardiac tissue.

A typical anchoring device comprises anchoring members in the shape of tines which, when placing the implantable medical device on cardiac tissue, pierce the cardiac tissue and in this way provide for such an active anchoring of the implantable medical device on the cardiac tissue. For placing the implantable medical device on cardiac tissue, the implantable medical device for example is received within a sheathing device of a delivery catheter, the delivery catheter being used to access the human heart and to deliver the implantable medical device towards a location of interest by removing the sheathing device and by in this way placing the implantable medical device on cardiac tissue at the location of interest. Upon delivering the implantable medical device from the sheathing device the anchoring members engage with cardiac tissue to provide for a fastening of the implantable medical device on the cardiac tissue.

US 9,844,659 describes an assembly of an implantable medical device comprising an anchoring device having a set of active fixation tines. The active fixation tines in the set are deployable from a spring-loaded position in which distal ends of the fixation tines point away from the implantable medical device to a hooked position in which the active fixation tines bend back towards the implantable medical device.

US 9,526,891 discloses an implantable pacemaker system including a housing having a proximal end and a distal end, an arrangement of anchoring members being placed on the distal end of the housing for providing for a fastening of the implantable pacemaker system on cardiac tissue.

US 8,700,181 discloses a leadless intra-cardiac medical device configured to be implanted entirely within a heart of a patient, the device including a housing configured to be securely attached to an interior wall portion of a chamber of the heart, and a stabilizing intra-cardiac device extension connected to the housing. The stabilizing intra-cardiac device extension may include a stabilizer arm or an appendage arm, or an elongated body or a loop member configured to be passively secured within the heart.

A typical implantable medical device such as a leadless pacemaker device is to be placed in a ventricle of the heart and is anchored on a ventricular wall. Tines of an anchoring device for this may pierce a portion of the ventricular wall and may hence fasten the implantable medical device on the ventricular wall, wherein damage to the ventricular wall due its thickness and strength typically is limited. If an implantable medical device however shall be placed in the atrium, a fixation needs to be provided on an atrial wall, taking into account that the atrial wall generally has a reduced strength and delicate structure and hence is more susceptible to being damaged.

US 2018/0126179 A1 discloses an implantable medical device which may be employed within a patient's right atrium at a location near a right atrial appendage of the patient's heart in order to pace the patient's heart and/or to sense electrical activity within the patient's heart. The implantable medical device includes an expandable anchoring mechanism configured to secure the implantable medical device in place for example within the right atrial appendage.

It is an objective of the instant disclosure to provide an implantable medical device and a method for implanting an implantable medical device which allow for a reliable and secure fastening of an implantable medical device at a location of interest for example in an atrium of a patient's heart.

### SUMMARY

This objective is addressed by an implantable medical device comprising the features of claim 1.

In one aspect, an implantable medical device comprises a housing and an anchoring device arranged on the housing, the anchoring device being configured to anchor the implantable medical device on intra-cardiac tissue. The anchoring device comprises an arrangement of elastic struts for abutment with intracardiac tissue, each strut comprising a first end and a second end, the struts being connected to the housing at their first ends. The anchoring device further comprises a connection structure connecting at least some of the struts of the arrangement of struts at their second ends.

The implantable medical device shall be implanted into a patient's heart such that it comes to rest for example within an atrium of the heart, in particular the right atrium. For securing the implantable medical device on intracardiac tissue, herein, the anchoring device shall provide for an anchoring for example on an atrial wall bounding the atrium towards the outside, wherein due to the structure of the atrial wall, in particular its reduced strength and thickness in comparison e.g. to a ventricular wall, the anchoring device is shaped such that an anchoring may be provided despite the specific delicate structure of the atrial wall.

The implantable medical device in particular may be an active implantable medical device and for this may comprise electronic circuitry as well as an energy supply such that the implantable medical device, once implanted into the heart, may perform a control and/or monitoring function within the heart, such as a pacing function and/or a sensing function. The implantable medical device in particular may be a leadless pacemaker device configured for implantation into an atrium of the patient's heart, in particular the right atrium of the patient's heart.

In one embodiment, the implantable medical device comprises an electrode arranged on the housing. The implantable medical device may in particular have the shape of a longitudinal capsule having a distal end and a proximal end, the implantable medical device being placeable on tissue within the patient's heart with the distal end. The housing herein encapsulates electric and electronic components of the device in a fluid-tight manner, the electrode being placed distally on the housing, wherein on the proximal end the housing may for example comprise a coupling device which may be used to establish a coupling to a delivery catheter or the like. The electrode serves to transmit and/or receive electrical signals and for this, when the medical device is implanted into the heart, is in abutment with cardiac tissue, i.e. the atrial wall on which the implantable medical device is anchored by the anchoring device.

The anchoring device is formed by a multiplicity of struts, particularly at least deformable, preferably elastic struts, which are shaped to abut with intracardiac tissue once the implantable medical device is placed within the patient's heart at a location of interest. The struts may generally expand from a collapsed position to transition towards an expanded position in which the struts may abut with intracardiac tissue, wherein the abutment may occur under an elastic tensioning and deformation of the struts.

The abutment of the struts with intracardiac tissue may in particular be atraumatic. In particular, no piercing structures such as tines, spikes, barbs or hooks may be required for providing for an anchoring by means of the elastic struts, wherein this does not exclude that additional piercing structures such as tines, spikes, barbs or hooks are arranged on the elastic struts or on other features of the anchoring device for providing for an additional interconnection of the anchoring device to intracardiac tissue.

The struts in particular may cause an elastic tensioning for positioning the implantable medical device in a patient's heart, wherein the tensioning may act to support a proper placement of the implantable medical device on intracardiac tissue. A tensioning provided by the anchoring device in particular may cause the electrode to be tensioned to contact with intra-cardiac tissue, such that proper operation of the electrode for emitting electrical signals into a conduction system of the heart or for receiving electrical signals for the purpose of sensing a cardiac activity is facilitated.

The struts of the anchoring device are at least partially connected by the connection structure at the second ends of the struts. By means of the connection structure a relative placement and tensioning of the struts with respect to each other may be provided, the struts being interconnected by the connection structure such that a coherent structure is established for abutment with intra-cardiac tissue and for providing a defined tensioning force on the implantable medical device.

In one embodiment, the housing has a generally longitudinal shape extending along a longitudinal axis, the housing comprising a distal end which is configured and intended to be placed on intra-cardiac tissue and a proximal end opposite the distal end. The struts herein, with their first ends, are for example connected to the housing at the proximal end, such that the struts extend from the proximal end of the housing generally towards the distal end.

The multiplicity of struts may be circumferentially distributed about the longitudinal axis, wherein the elastic struts may be arranged on the housing equidistantly or non-equidistantly with respect to each other when viewed along a circumferential direction about the longitudinal axis.

In one embodiment the struts extend radially outside of the housing from the proximal end such that the second ends of the struts are placed radially apart from the housing. The struts herein may assume a curved shape and may generally extend from the housing in a predominantly radial direction, in a predominantly longitudinal direction or in an angled direction corresponding to a superposition of a radial vector component and a longitudinal vector component. An anchoring of the implantable medical device on intracardiac tissue hence may be provided by means of the anchoring device in the vicinity of the proximal end of the housing and circumferentially about the housing, which in particular may facilitate a placement and anchoring of the implantable medical device within a confined location within the patient's heart, for example within the right atrial appendage or the left atrial appendage.

In one embodiment, the connection structure comprises a connector strut which extends in between at least a portion of the struts to connect said portion of the struts at their second ends. The connector strut may in particular, in one embodiment, connect all struts to each other at their second ends, the connector strut for this forming a ring that circumferentially extends in between the second ends of the struts and hence interconnects the second ends of the struts to each other.

The struts of the arrangement of struts may for example be formed from an elastic material, such as a metal material, for example a shape memory alloy, such as a nickel titanium alloy, for example Nitinol. Alternatively, the struts may be formed from a biocompatible plastics or silicone material, for example a polymer material such as PEEK.

Likewise, the connector strut of the connection structure may be formed from an elastic material, such as a metal material, for example a shape memory alloy, such as a nickel titanium alloy, for example Nitinol. Alternatively, the connector strut may be formed from a biocompatible plastics or silicone material, for example a polymer material.

In one embodiment, the anchoring device comprises a planar, flat or areal structure extending, particularly flatly, across, particularly encircling at least a group of the struts. The anchoring device hence forms an umbrella-like configuration in which a material arrangement extends in between a support structure formed by the struts and the connection structure. The planar structure herein may be flexibly deformable and may be spanned by the support structure formed by the struts and the connection structure, wherein the planar structure may for example be a membrane formed from a biocompatible material, such as a plastics or silicone material, a bio-compatible mesh or a biocompatible woven fabric.

Generally, the planar structure may be formed from a weave or other structure of a bio-resorbable or non-resorbable material.

The planar structure may extend across all struts, or over a sub-group of the entirety of the struts. The planar structure beneficially is permeable for fluids, but also may be non-permeable.

In one embodiment, the planar structure comprises one or multiple adhesion features for adhering to intracardiac tissue. Such adhesion features for example may be formed by pockets of biocompatible glue which may for example be rapidly polymerizing upon contact with tissue and may provide for an adhesion of the anchoring device to intra-cardiac tissue.

Alternatively or in addition, the adhesion features may for example be formed by positive-locking connection features such as tines, barbs, spikes or hooks arranged on the structure formed by the struts, the connection structure and the planar structure.

In one embodiment, the anchoring device is collapsible to assume a collapsed position for implantation of the implantable medical device using a delivery device, such as a delivery catheter. The anchoring device herein is expandable from the collapsed position to abut with intra-cardiac tissue upon deployment from the delivery device. The anchoring device with its struts, the connection structure and also, potentially, a planar structure arranged on the support structure formed by the struts and the connection structure is collapsible such that it may be received on a delivery device in a collapsed position in which the radial extension of the anchoring device is reduced. Once the implantable medical device is deployed from the delivery device the anchoring device may expand such that the struts may abut with intra-cardiac tissue and hence may provide for a positioning and fixation of the implantable medical device within a chamber of the heart.

The anchoring device may be configured to self-expand once the implantable medical device is deployed from the delivery device and hence the anchoring device no longer is radially confined within an associated receptacle of the delivery device. For this, the anchoring device with its struts and the connection structure may transition towards a preset shape once the implantable medical device is deployed from the delivery device.

Alternatively or in addition, the expansion of the anchoring device from the collapsed position to the expanded position may be supported by means of an expansion device, the expansion device being actuatable, for example using the delivery device, to act onto the struts for expanding the anchoring device. Alternatively, the expansion of the anchoring device from the collapsed position to the expanded position may be actuated by means of an inflatable structure, e.g. an inflatable balloon or pocket, which may be part of the anchoring device, the implantable medical device or the delivery system (e.g. catheter). For example, the anchoring device is provided with sufficient opening for the, e.g. ballons to be pulled back through after expansion and deflation, respectively. The expansion device may for example comprise expansion members interconnected with the struts and with an adjustment member which is movably arranged on the housing. Hence, by adjusting the position of the adjustment member with respect to the housing and expansion force may be introduced into the struts such that the struts are caused to deform towards their expanded position.

In another aspect, a method for implanting an implantable medical device into a patient's heart is provided, the method comprising: introducing the implantable medical device comprising a housing into a chamber of the patient's heart; and anchoring the implantable medical device on intra-cardiac tissue within the chamber using an anchoring device arranged on the housing; wherein said anchoring includes: causing an expansion of an arrangement of elastic struts of the anchoring device to abut with intra-cardiac tissue, each strut comprising a first end and a second end, the struts being connected to the housing at their first ends, wherein the anchoring device further comprises a connection structure connecting at least some of the struts of the arrangement of struts at their second ends.

The advantages and advantageous embodiments described above for the implantable medical device equally apply also to the method, such that it shall be referred to the above in this respect.

The advantages and advantageous embodiments described above for the implantable medical device equally apply also to the method, such that it shall be referred to the above in this respect.

The various features and advantages of the present invention may be more readily understood with reference to the following detailed description and the embodiments shown in the drawings. Herein,
- Fig. 1: shows a schematic view of the human heart;
- Fig. 2: shows a schematic drawing of an implantable medical device in the shape of a leadless pacemaker device, the implantable medical device having a housing and an anchoring device arranged thereon;
- Fig. 3: shows a schematic view of the embodiment of the implantable medical device of Fig. 2 when being placed on intracardiac tissue;
- Fig. 4: shows a schematic view of the implantable medical device when received for example within a confined chamber such as the right atrial appendage of the heart;
- Fig. 5: shows a modified embodiment of the implantable medical device;
- Fig. 6: shows a schematic view of yet another embodiment of an implantable medical device;
- Fig. 7: shows yet another embodiment of an implantable medical device;
- Fig. 8: shows yet another embodiment of an implantable medical device;
- Fig. 9: shows a schematic view of an embodiment of an implantable medical device received within a delivery device for implanting the implantable medical device within a patient's heart; and
- Fig. 10: shows the implantable medical device together with the delivery device upon deployment of the implantable medical device.

Subsequently, embodiments shall be described in detail with reference to the drawings. In the drawings, like reference numerals designate like structural elements.

It is to be noted that the embodiments are not limiting for the instant disclosure, but merely represent illustrative examples.

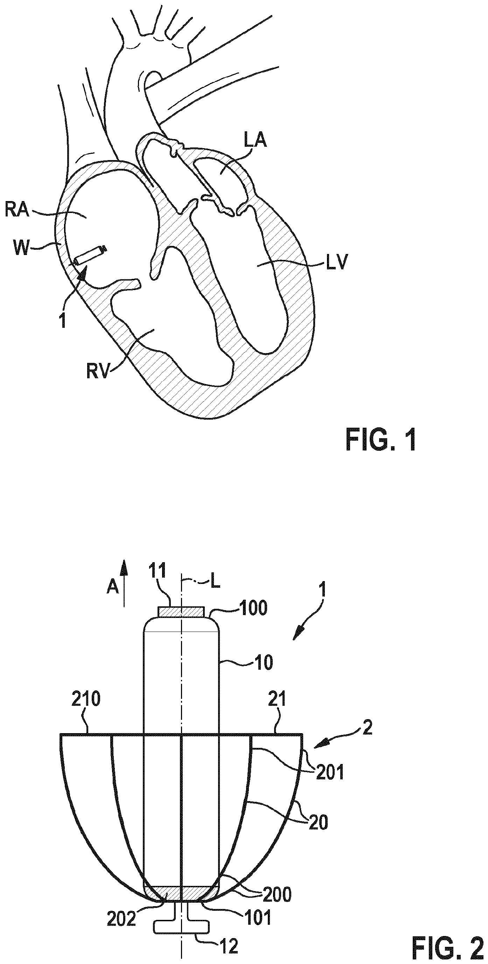

Fig. 1 shows, in a schematic drawing, a human heart comprising a right atrium RA, a right ventricle RV, a left atrium LA and a left ventricle LV, an implantable medical device 1 being implanted for example into the right atrium RA, the implantable medical device 1 e.g. having the shape of a leadless pacemaker device for providing a pacing of the heart's activity at the right atrium RA.

For implantation the implantable medical device 1 by means of a delivery system is placed e.g. in the right atrium RA and is delivered from the delivery system such that it comes to rest on intra-cardiac tissue e.g. on an atrial wall W and is fastened to intracardiac tissue.

Fig. 2 shows, in a schematic drawing, an example of an implantable medical device 1 in the shape of a leadless pacemaker device, the implantable medical device 1 having a housing 10 forming a proximal end 101 and a distal end 100, the implantable medical device 1 being configured to be placed on intracardiac tissue, for example an atrial wall W, by means of its distal end 100. On the distal end 100, herein, an electrode 11 is placed which, potentially together with other electrodes of the implantable medical device 1, serves to provide for a pacing action in order to stimulate cardiac activity in a defined manner.

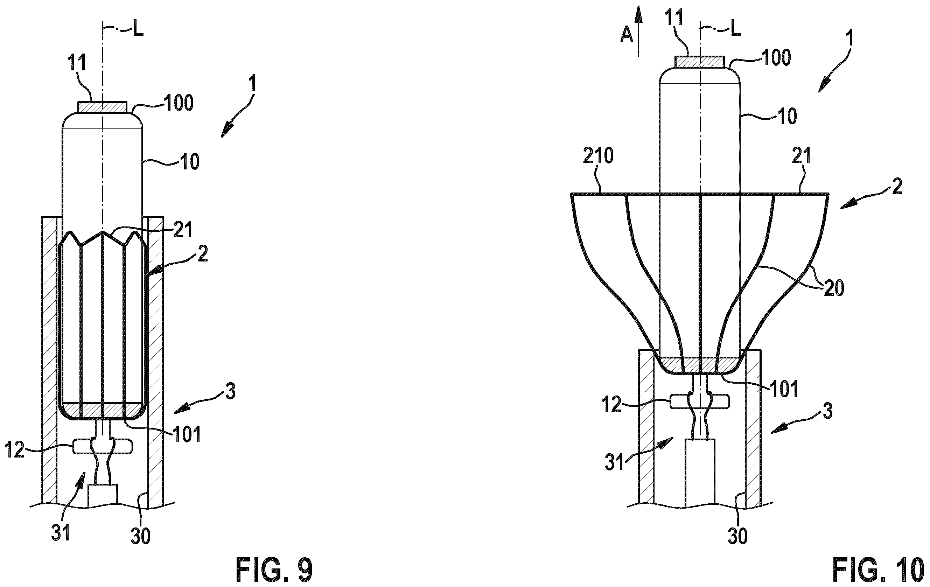

The implantable medical device 1 has the shape of a capsule extending longitudinally along a longitudinal axis L, the housing 100 encapsulating electrical and electronic components of the implantable medical device 1 therein in a fluid-tight manner. Whereas the distal end 100 with the electrode 11 arranged thereon is to be placed on and shall abut with intra-cardiac tissue for operation of the implantable medical device 1, the proximal end 101 carries a coupling device 12 which provides for a coupling of the implantable medical device 1 to a delivery device during implantation of the implantable medical device 1, as this schematically is shown in Figs. 9 and 10.

During implantation the implantable medical device 1 is to be placed on intra-cardiac tissue, e.g. an atrial wall W, and is to be fastened to intracardiac tissue. For this, the implantable medical device 1 comprises an anchoring device 2, which shall provide for an anchoring to intracardiac tissue to ensure a proper fixation of the implantable medical device 1.

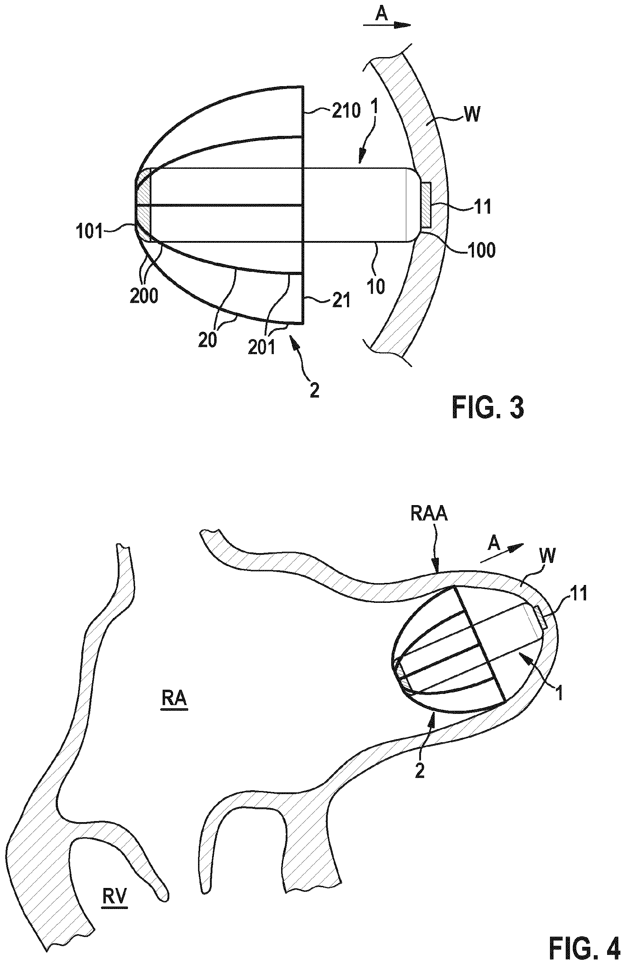

Referring now to Figs. 2 and 3, the anchoring device 2 in one embodiment comprises a multiplicity of struts 20 which each at a first end 200 are connected to a base 202 and, by means of the base 202, to the housing 10 at its proximal end 101. The base 202 may for example have the shape of a ring extending circumferentially about the housing 10 at the proximal end 101 or a cap being placed on the proximal end 101. Alternatively, the base 202 may be integrated with the housing 10 such that the struts 20 are integrally connected with the housing 10 at the ends 200 of the struts 20.

The struts 20 of the anchoring device 2 generally extend from the proximal end 101 of the housing 10 towards the distal end 100 and for this are placed radially outside of the housing 10, the struts 20 being circumferentially distributed about the generally cylindrical housing 10 such that neighboring struts 20 have equal distances along the circumference of the housing 10.

In particular, the struts 20 extend radially towards the outside and, in a superimposed manner, axially along the longitudinal axis L towards the proximal end 100 of the housing 10, the struts 20 at second ends 201 being placed radially apart from the cylindrical housing 10.

At their ends 201 the struts 20 are interconnected with each other by means of a connection structure 21 having a connector strut 210 which in a ring-like fashion circumferentially extends about the housing 10 and interconnects all ends 201 of the struts 20 with each other.

The connector strut 210 may be integrally formed with the struts 20, for example by cutting, in particular laser cutting the entire assembly of the anchoring device 2 from a single piece of material, in particular a metal material, or may be connected to the struts 20 at the ends 201 for example by a welding or crimping connection. Alternatively, the connector strut 210 and the struts may be joined by textile-like connections with loops etc. or being braided or knitted.

The connection structure 21 serves to connect the struts 20 to each other at the ends 201. Hence, the arrangement of the struts 20 operatively acts together for positioning the implantable medical device 1 on intracardiac tissue, in particular for providing a tensioning with respect to intracardiac tissue for properly positioning and holding in place the implantable medical device 1 with respect to intracardiac tissue.

In particular, as illustrated in Fig. 4, by means of the anchoring device 2 the implantable medical device 1 may be anchored within a confined chamber of the heart, for example within the right atrial appendage RAA of the right atrium RA of the heart. Herein, the anchoring device 2 by means of its struts 20 abuts surrounding intracardiac tissue and provides for a tensioning of the implantable medical device 1 with respect to the surrounding tissue, such that a tensioning force in an abutment direction A along the longitudinal axis L of the implantable medical device 1 is provided to push the electrode 11 arranged at the distal end 100 of the housing 10 into tight contact with intra-cardiac tissue, in particular the atrial wall W.

The anchoring device 2 with its curved struts 20 and the connection structure 21 is shaped such that the anchoring device 2 abuts with intracardiac tissue in an atraumatic fashion, hence without piercing or otherwise damaging the intra-cardiac tissue. By means of the anchoring device 2 the implantable medical device 1 hence may be tightly anchored on thin and delicate structures, for example the atrial wall W, within the patient's heart.

The struts 20 are formed elastic and in particular may be collapsed towards the housing 10 to be received in a delivery device 3, as this is illustrated in Figs. 9 and 10.

For delivery the implantable medical device 1 is received within an inner lumen 30 of the delivery device 3 in the shape of a delivery catheter, as shown in Fig. 9. The anchoring device 2 for this is radially compressed such that the struts 20 are approached towards the housing 10 of the implantable medical device 1, under elastic deformation of the struts 20 and the connection structure 21. By means of a coupling assembly 31 of the delivery device 3 an operative connection to the implantable medical device 1 is established.

As illustrated in Fig. 10, for deploying the implantable medical device 1 the delivery device 3 is retracted such that the implantable medical device 1 is unsheathed and the anchoring device 2 is free to radially expand to assume an expanded position. The anchoring device 2 by means of the struts 20 and the connection structure 21 hence may abut with intra-cardiac tissue for positioning the implantable medical device 1 at a location of interest, the anchoring device 2 beneficially providing for a back force to push the implantable medical device 1 with its electrode 11 arranged on the distal end 100 of the housing 10 into tight contact with tissue, in particular into a receiving feature such as a distal tip of the right atrial appendage RAA, as this is illustrated in Fig. 4.

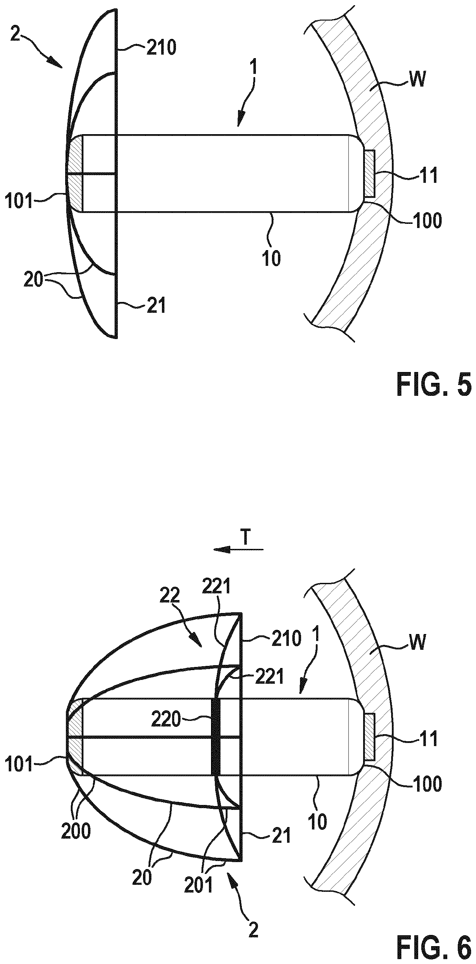

In a modified embodiment, shown in Fig. 5, the anchoring device 2 comprises a more radial extension, struts 20 of the anchoring device 2 pointing radially outwards, but having a reduced axial extension in comparison to the embodiment of Figs. 2 and 3.

The anchoring device 2, in principle, may be shaped such that a beneficial abutment with a particular anatomic feature within the patient's heart may be achieved.

In the embodiments of Figs. 2 to 5 the anchoring device 2 may be self-expandable in that the anchoring device 2 resumes to a preset shape corresponding to an expanded position once it is deployed from a delivery device 3, as visible from Figs. 9 and 10.

Referring now to Fig. 6, in another embodiment the anchoring device 2 comprises struts 20 and a connection structure 21 similar to the one as described for the embodiment of Figs. 2 and 3, wherein in addition an expansion device 22 is provided which serves to support an expansion of the anchoring device 2 from a collapsed position in order to bring the struts 20 into abutment with surrounding tissue for positioning the implantable medical device 1 within the patient's heart.

The expansion device 22 comprises tensioning members 221 which at one end are connected to the struts 20 and at another end are placed on an adjustment member 220 which is movable with respect to the housing 10 and may be actuated in order to support a radial expansion of the anchoring device 2.

In particular, in the embodiment of Fig. 6 the adjustment member 220 may be actuated, for example by means of a suitable actuation mechanism of the delivery device 3, to be displaced on the housing 10 by a sliding movement of the adjustment member 220 in a tensioning direction T towards the proximal end 101 of the housing 10. In this way a tensioning force may be applied to the struts 20, such that the struts 20 for example are forced to bend and to in this way radially expand for abutting with surrounding tissue.

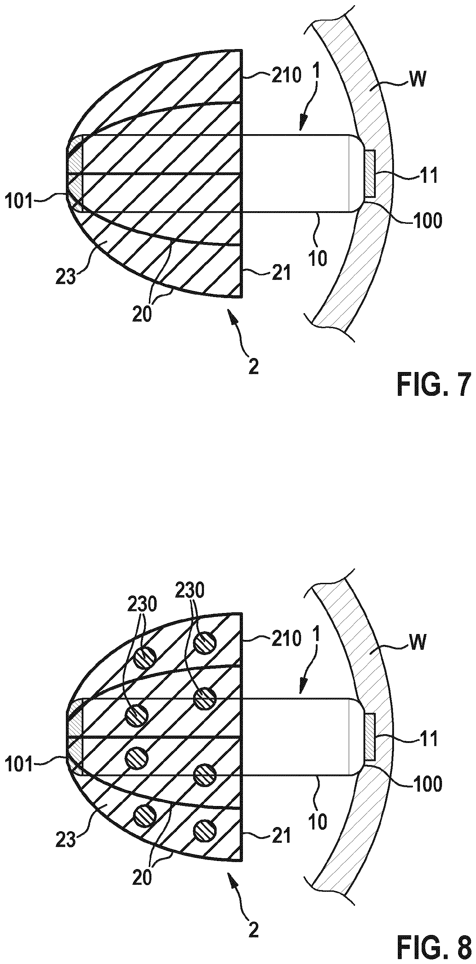

Referring now to Fig. 7, in one embodiment the struts 20 of the anchoring device 2 comprise a planar structure 23 in the shape of a membrane, a mesh or a woven or non-woven fabric which spans across the struts 20 of the anchoring device 2 and hence provides for a circumferential coverage across the struts 20. The anchoring device 2 hence assumes an umbrella-like configuration in its expanded position, wherein free spaces in between the struts 20 are spanned by the planar structure 23, which is formed from a biocompatible material and may be permeable in order not to block a fluid passage within the patient's heart.

Referring now to Fig. 8, in a modified embodiment of the embodiment of Fig. 7 adhesion features 230 for example in the shape of pockets containing a biocompatible glue are provided on the planar structure 23, the adhesion features 230 being formed to come into contact with tissue upon placement of the implantable medical device 1 in the patient's heart and to a tier to intra- in order to provide for an additional fixation of the implantable medical device 1 within the patient's heart. Alternatively or additionally, the planar structure may comprise electrodes, particularly configured to sense physiological electrical signals from the tissue.

The adhesion features 230 may be regularly or irregularly distributed across the planar structure 23. The adhesion features 230 may comprise a glue material, or alternatively may be implemented by physical interconnection features such as tines, spikes, barbs or hooks for engaging with surrounding tissue.

The struts 20 and the connector strut 210 of the connection structure 21 in all embodiments may be formed from a deformable or elastic material, such as a biocompatible polymer, such as silicone or PEEK. Alternatively, the struts 20 and the connector strut 210 of the connection structure 21 in all embodiments may be formed from an elastic metal material, for example a shape memory alloy, for example a nickel titanium alloy, for example Nitinol.

The anchoring device 2 may be integrally formed as one part, for example by cutting the structures of the anchoring device 2 from a single piece of material, or by integrally forming the anchoring device 2 using a suitable molding or additive forming technique. Alternatively, the anchoring device 2 may be formed from multiple parts which are connected to each other for example by welding or crimping connections or alternative by textile-like connections such as braiding or knitting.

An anchoring device as described herein may provide for an easy deployment.

A correct deployment herein may easily be verified by visualization using for example a suitable imaging technique, such as an X-ray technique, the deployment being recognizable by a proper expansion of the anchoring device.

An anchoring device as described herein may be compatible with thin and delicate tissue structures, such as the atrial wall, while providing a secure fixation with the possibility of a tensioning of the implantable medical device on the tissue.

### LIST OF REFERENCE NUMERALS

- 1: Leadless pacemaker device
- 10: Housing
- 100: Distal end
- 101: Proximal end
- 11: Electrode
- 12: Coupling device
- 2: Anchoring device
- 20: Struts
- 200,201: End
- 202: Base member
- 21: Connection structure
- 210: Connector strut
- 22: Expansion device
- 220: Adjustment member
- 221: Tensioning member
- 23: Planar structure (substrate, fabric or membrane)
- 230: Adhesion feature
- 3: Delivery device
- 30: Lumen
- 31: Coupling assembly
- A: Abutment direction
- L: Longitudinal axis
- LA: Left atrium
- LV: Left ventricle
- RA: Right atrium
- RAA: Right atrial appendage
- RV: Right ventricle
- T: Tensioning direction
- W: Atrial wall

## Claims

1. An implantable medical device (1), comprising:
a housing (10); and
an anchoring device (2) arranged on the housing (10) and being configured to anchor the implantable medical device (1) on intra-cardiac tissue,
wherein the anchoring device (2) comprises an arrangement of at least deformable, particularly elastic, struts (20) for abutment with intracardiac tissue, each strut (20) comprising a first end (200) and a second end (201), the struts being connected to the housing (10) at their first ends (200),
wherein the anchoring device (2) further comprises a connection structure (21) connecting at least some of the struts (20) of the arrangement of struts (20) at their second ends (201).

2. The implantable medical device (1) according to claim 1, comprising an electrode (11) being arranged on the housing (10), the electrode (11) being configured to abut intracardiac tissue for at least one of emitting an electrical signal and receiving an electrical signal.

3. The implantable medical device (1) according to claim 1 or 2, wherein the housing (10) has a generally longitudinal shape extending along a longitudinal axis (L), the housing (10) comprising a distal end (100) which is configured and intended to be placed on intracardiac tissue and a proximal end (101) opposite the distal end (100).

4. The implantable medical device (1) according to claim 3, wherein the struts (20) with their first ends (200) are connected to the housing (10) at the proximal end (101).

5. The implantable medical device (1) according to claim 3 or 4, wherein the struts (20) extend radially outside of the housing (10) such that the second ends (201) of the struts (20) are placed radially apart from the housing (10).

6. The implantable medical device (1) according to one of claims 3 to 5, wherein the struts (20) extend along at least one of a radial direction and a longitudinal direction with respect to the longitudinal axis (L) of the housing (20).

7. The implantable medical device (1) according to one of the preceding claims, wherein the connection structure (21) comprises a connector strut (210) which extends in between at least a portion of the struts (20) to connect said portion of the struts (20) at their second ends (201).

8. The implantable medical device (1) according to claim 7, wherein the connector strut (210) is formed as a ring connecting all struts (20) of the arrangement of struts (20) at their second ends (201).

9. The implantable medical device (1) according to one of the preceding claims, wherein the anchoring device (2) comprises a planar structure (23) extending, particularly flatly, across at least a group of the struts (20).

10. The implantable medical device (1) according to claim 9, wherein the planar structure (23) comprises a membrane, fabric or mesh extending, particularly flatly, across said group of the struts (20).

11. The implantable medical device (1) according to claim 9 or 10, wherein the planar structure (23) comprises at least one adhesion feature (230) for adhering to intra-cardiac tissue, and/or one or more electrodes, particularly configured to sense physiological electric signals.

12. The implantable medical device (1) according to one of the preceding claims, wherein the anchoring device (2) is collapsible to assume a collapsed position for implantation of the implantable medical device (1) using a delivery device (3), wherein the anchoring device (2) is expandable from the collapsed position to abut with intra-cardiac tissue upon deployment from the delivery device (3).

13. The implantable medical device (1) according to claim 12, wherein the anchoring device (2) comprises an expansion device (22) operatively connected to the struts (20) and being actuatable for expanding the anchoring device (2) towards the expanded position.

14. The implantable medical device (1) according to claim 13, wherein the expansion device (22) comprises an adjustment member (220) movably arranged on the housing (10).

15. A method for implanting an implantable medical device (1) into a patient's heart, comprising:
introducing the implantable medical device (1) comprising a housing (10) into a chamber of the patient's heart; and
anchoring the implantable medical device (1) on intra-cardiac tissue within the chamber using an anchoring device (2) arranged on the housing (10);
wherein said anchoring includes: causing an expansion of an arrangement of at least deformable, particularly elastic, struts (20) of the anchoring device (2) to abut with intra-cardiac tissue, each strut (20) comprising a first end (200) and a second end (201), the struts (20) being connected to the housing (10) at their first ends (200), wherein the anchoring device (2) further comprises a connection structure (21) connecting at least some of the struts (20) of the arrangement of struts (20) at their second ends (201).
